# EUROPEAN PATENT APPLICATION

(11) **EP 2 765 839 A1**
(43) Date of publication of application: **13.08.2014**
(21) Application number: 14164439.3
(22) Date of filing: 28.02.2006
(51) Int. Cl.: H05K 1/00, A61B 5/00, G08B 21/00, A61F 13/15, G08B 23/00

(54) **Flexible electronic device**

(30) Priority: 28.02.2005 AU 2005900931
(62) Divisional of application: 06704928.8
(71) Applicant: Commonwealth Scientific and Industrial Research Organisation, Campbell, Australian Capital Territory 2612 (AU)
(72) Inventor: Mestrovic, Michael, Moriac, Victoria 3240 (AU); D'Arcy, Brendan, Grovendale, Victoria 3216 (AU); Helmer, Richard, Geelong West, Victoria 3218 (AU); Humphries, Bill, Newtown, Victoria 3220 (AU)
(74) Representative: Finnie, Peter John

(57) **Abstract**

A device that may be worn by a person, or fitted to an apparatus for monitoring, sensing or controlling one or more parameters such as, but not limited to, moisture, temperature, heart rate or respiration rate, or controlling various parameters of electronic functions. The device includes; a) a flexible substrate (10) that can be worn by a person or fitted to an apparatus and wherein the flexible substrate requires disposal or replacement with a fresh flexible substrate or requires maintenance that prevents continuous use of the device. b) An electrical circuit having separate first (11) and second (16) parts, where the first part (11) is carried by the flexible substrate (10) and the first (11) and second parts (16) can be connected together to form an electrical connection (13,14) and disconnected to allow the parts to be reused or disposed as desired.

## Description

### FIELD AND BACKGROUND OF THE INVENTION

The present invention relates to a device that may be worn by a person or fitted to an apparatus and includes any one or a combination of the following: a sensor for sensing or measuring a parameter, a control interface for controlling a parameter, or a monitoring interface for monitoring a parameter. The parameter may be any condition, property or state including environmental or operating parameters, or vital signs of life. In addition, the parameter may be any operating parameter of an electronic device.

For example, in the situation where the device of the present invention is in the form of a disposable diaper or nappy, the device may include a sensor adapted for signalling when the nappy or diaper requires changing.

According to another example, the device of the present invention may be in the form of a singlet, vest, or T-shirt including a sensor that is adapted for monitoring the heart rate and/or respiration rate of a patient, athlete, or person wearing the device.

According to another example, the device of the present invention may be in the form of a textile including a control interface with single or multiple switches or buttons that control the function of an electronic device. In the case where the electronic device is in the form of a radio or media player, the control interface may be used for controlling an operating parameter such as the volume of sound played by the device.

According to yet another example, the device of the present invention may be in the form of a belt including a sensor that can be fitted to an apparatus. The sensor may be adapted for use as either an impact or strain gauge for evaluating the forces applied to the apparatus or recording the amount of work performed by the apparatus.

There are a large number of devices presently available that are capable of sensing, controlling, or monitoring various parameters such a moisture, strain, and heart rate or the operating parameter of an electronic device. However, in most cases the cost of manufacture of the devices requires that the devices be reused a number of times.

### SUMMARY OF THE INVENTION

One of the aims of the present invention is to provide a device that includes an electrical circuit having any one or a combination of a sensor, or monitor interface, or control interface and where the electrical circuit has two or more parts which a user can connect or disconnect to facilitate reuse or disposal of the parts of the circuit as desired.

In particular, according to the present invention there is provided a device that may be worn by a person, or fitted to an apparatus for monitoring, sensing or controlling one or more parameters such as, but by no means limited to, moisture, temperature, heart rate or respiration rate, or controlling various parameters of electronic functions. The device includes:
a) a flexible substrate that can be worn by a person or attached to an apparatus and, wherein the flexible substrate requires disposal and replacement with a fresh flexible substrate or requires maintenance that prevents continuous use of the device; and
b) an electrical circuit having separate first and second parts, wherein the first part is carried by the flexible substrate and the first and second parts can be connected together to form an electrical connection and thereafter disconnected to allow the parts to be reused or disposed as desired, and wherein one or both of the first and second parts includes any one or combination of a controller for controlling a parameter, a monitor for monitoring a parameter or a sensor for sensing a parameter.

An advantage provided by the present invention is that it enables the interchangeability, assembly and reassembly of the first and second parts of the electrical circuit in a "transient" manner. In particular, an advantage provide by the first and second parts of the circuit being disconnectable is that when the flexible substrate requires disposal or maintenance such as washing, the second part can be disconnected and reused once the flexible substrate has been substituted with a fresh substrate. The electronic components included in the first and second parts of the circuit can be configured to suit particular applications and, therefore, whether the flexible substrate will be washed or disposed.

Throughout this specification the terms "flexible" or "flexible substrate" embrace any material that can change in shape or configuration. It is ideal, but by no means mandatory or compulsory that the change in shape or configuration can be the result of incidental forces applied to the material and that the changes in shape and configuration of the material are not permanent or irreversible. Examples of flexible substrates include but are by no means limited to: textile material and nontextile material such as plastic and board material that can flex without having drape properties traditionally possessed by textiles.

Although it is possible that the first part of the circuit may be indirectly attached to flexible substrate, it is preferred that the first part of the circuit be directly attached to the flexible substrate.

It is preferred that the first part of the circuit be able to flex without retarding the flexibility of the flexible substrate.

It is also preferred that the first part of the circuit be formed on or embedded in the flexible substrate using electronic circuit printing techniques and or textile processing techniques which enable the incorporation of conductive materials, such as conductive filaments and yarns, by knitting, weaving, and layering, and/or combinations of these techniques.

It is possible that the device may also include a second substrate, preferably in the form of a second flexible substrate and that the second part of the circuit be carried by the second flexible substrate. During use, the first or second substrate may require washing or substitution with a fresh substrate. For example, in the situation where the first substrate is due for washing or disposal, it is envisaged that the second part of the circuit will include the more valuable component of the electrical circuit.

It is preferred that the device be in the form of a textile and that the flexible substrate on which the first part of the circuit is carried be incorporated in the textile. In this situation and when the first part of the circuit includes a sensing element and/or control interface, an advantage provided by flexible substrate being incorporated into a textile or garment is that the sensing element can sense parameters very close to a person's skin such as surface temperature, skin conductivity and moisture and/or provide an intimate monitor interface and or control interface for controlling electronic devices.

The term "textile or garment" embraces any form of garment that may be worn by a person including jumpers, sweaters, vests, pants, T-shirts, underwear, belts, gowns, personal flotation devices, safety apparel, nappies and diapers, and any other form of textile such as that used during road construction, sun shade or solar protection, and wrapping.

Although it is possible that the first and second parts can be held together in electrical connection by any suitable means including clips, clasps, **Velcro∼** and other co-operating male and female members, it is preferred that a releasable conductive adhesive bond the first and second parts of the circuit together.

The adhesive may also be used with one or a combination of the other types of mechanisms mentioned in the preceding paragraph for holding the first and second parts together.

Any suitable adhesive that resists accidental separation of the first and second parts of the circuit yet allows the first and second parts to be manually separated would be suitable. In other words, according to one preferred form of the invention, it is envisaged that the adhesive provide a basis for a transient electrical connection between the first and second parts of the circuit.

It is preferred that the first part of the circuit include a first pair of contact surfaces and that the second part of the circuit include a second pair of co-operating contact surfaces, and that the first and second pairs of the contact surfaces can be manually aligned and placed together to facilitate electrical connection therebetween. It is also preferred that the first and second parts be able to be manually disconnected so that either one or both of the parts can be reused.

In the situation where an adhesive is at least in part responsible for holding the first and second parts together in electrical connection, it is preferred that the adhesive be located between the first and second pairs of the contact surfaces and that the adhesive preferably be an electrically conductive adhesive so that an electrical path can be established between the first and second pairs of contact surfaces.

It is preferred that the electrical contact surface of each of the first or second part have a total surface area of greater than or equal to 1 square millimetre.

In the situation where an adhesive is located between the first and second pairs of contact surfaces when the electrical connection is made, it is preferred that the adhesive be pre-applied to one of the contact surfaces before electrical connection and that a removable protective tab be positioned to cover the adhesive on the contact surfaces and prevent the adhesive from bonding to other objects. In use, the protective tab can be removed just prior to the first and seconds part being placed together to form the electrical connection and can be replaced over the contact surfaces when the parts have been separated after use.

The electrical circuit may have any required electronic components including but not limited to:
- an electrical power source for powering the circuit;
- a processing unit for receiving information from the controller, monitor or sensor on the parameter being monitored, sensed or controlled and processing the information using suitable techniques, such as by comparing the information on the parameter to a preselected value or set of values of the parameter;

- a controller interface for allowing the on and off operation of the power source or control another operational function of the device;
- an output system for sending processed and/or unprocessed signals and/or for signalling when information on the parameter being controlled, monitored or sensed equals, exceeds or is less than a pre-selected value of the parameter being monitored, sensed or controlled.

Preferably, the transmitter includes a suitable antenna for transmitting a signal to a remote receiver that informs a person that a selected condition or parameter change has occurred and that action may be required. For example, in the situation where the garment is a disposable diaper and the electrical circuit has the capacity to transmit a signal to a centralised care station or care manager who may be caring for a number of persons, the device can then be used to identify when and on which persons need their nappies changed.

In the situation in which the flexible substrate forms part of a disposable diaper, pad, incontinency sanitary product or wound dressing, and the parameter being monitored, sensed or controlled is moisture and the first part of the circuit carried by the flexible substrate forms part of the garment and can be disposed and the second part of the circuit can be reused by removing it from the used garment and connecting it to a fresh garment. In order to reduce overall costs, it is envisaged that the second part which to be reused would be designed to include the more expensive components of the electrical circuit.

In the situation when the device is worn by a person and includes a sensor, the sensor may be adapted for sensing parameters such as, but by no means limited to, surface temperature, heart rate or respiration rate. In the situation when the device worn by a person includes a control interface in the form of one or more switches, the switches may form part of a media player and be used for controlling the volume or selection of music played.

In the situation where in the device is fitted to an apparatus or placed on an object, the sensor may sense one or more parameters such as but by no means limited to strain, temperature, moisture, light intensity, sound level, pressure and impact forces.

### BRIEF DESCRIPTION OF THE DRAWING

Two preferred embodiments of the present invention will now be described with reference to the accompanying drawings, of which:
Figures 1 and 2 illustrate perspective views of flexible and rigid substrates respectively according to an embodiment of a device of the present invention wherein the substrates are disconnected;
Figure 3 illustrates a perspective of the flexible and rigid substrates shown in Figures 1 and 2 connected together;
Figures 4 and 5 illustrates perspective views of two flexible substrates that are disconnected according to an alternative embodiment of a device of the present invention; and
Figure 6 illustrates a perspective view of the two flexible substrates shown in Figures 4 and 5 connected together.

### DETAILED DESCRIPTION

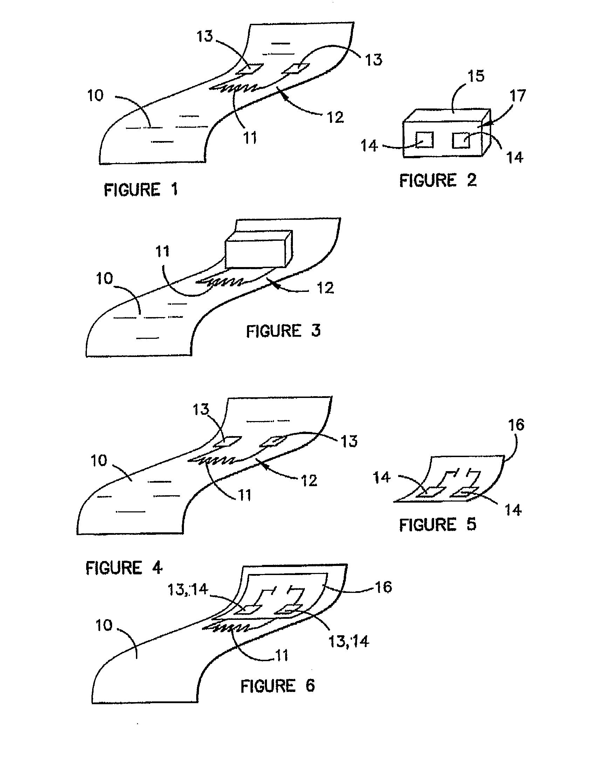

The embodiments of the invention shown in Figures 1 to 6 include a number of features in common and the.same reference numerals have been used to identify the same or similar features on both embodiments.

The embodiments of the invention shown in the Figures are described in the context of sensing moisture in disposable diapers, pads and alike sanitary products that are disposed or destroyed after use. However, it will be appreciated that the present invention is able to be used in the wide range of other applications such as protective or conventional clothing and covers that can be fitted to apparatuses. In the situation where the device of the invention forms part of a garment such as pants, a jacket or a shirt, it is envisaged that the device include electrical circuitry having at least two parts and that at least one part of the circuit is mounted on or in embedded in the textile material that makes up the garment. When the garment requires washing, the electrical circuit can be separated into parts such that only the washable component remains on the garment during washing. The electrical circuit may be adapted for sensing, monitoring or controlling parameters such as temperature, strain, movement of the garment or the operation of a device.

With reference to the embodiment of the invention shown in Figures 1 to 3, the device includes a flexible substrate identified by reference numeral 10 that forms part of a diaper and on which a first part 12 of an electrical circuit is embedded and/or printed using suitable techniques and technology. The first part 12 of the electrical circuit shown in Figure 1 includes ∼ conventional moisture sensor 11 for sensing bodily excretion indicating when the diaper needs to be changed.

The flexible substrate 10 may be any suitable material such as the liquid permeable material commonly used to manufacture diapers. It will be appreciated that the first part 12 of the electrical circuit need not be directly attached to the flexible substrate as shown in the Figures. For example, it is possible that the first part 12 of the electrical circuit carried by the flexible substrate may be directly attached to a rigid sheet which is bonded or laminated to the flexible substrate 10. However, to avoid the device from impeding the person's movement or reducing the comfort of the garment, it is preferred that the first part 12 of the circuit be directly attached or embedded in the flexible substrate 10 and not retard the flexibility of the substrate 10.

The first part 12 of the electrical circuit also includes two contact surfaces 13 in the form of electrodes for forming an electrical connection with corresponding electrodes 14 formed on the underside of the rigid substrate 15 shown in Figure 2. The rigid substrate 15 houses the remaining components of a second part 17 the electrical circuit including a power source, logic chip and output transmitter for sending signals to a receiver or a station of receivers operated by a manager caring for a number of patients. The first and second parts 12 and 17 of the electrical circuit form an operable circuit for sensing, control or monitoring a parameter.

Although not shown in the Figures, an electrical conductive adhesive is spread over the electrodes 13 and 14 of either one or a combination of the rigid or flexible substrates 10 and 15. The adhesive may also be located on the regions of the flexible substrate 10 adjacent to the electrodes and on the underside of the rigid substrate 15. In addition, a protective cover not shown in the Figures may be placed over the adhesive on either one or both of the substrates 10 and 15 to prevent removal of adhesive and prevent contamination of the adhesive by unwanted particles sticking to the adhesive.

An example of a suitable conductive adhesive is electrical conductive tape sold under the trade name ARcladm 8001 by Adhesive Research, Inc. Glen Rock PA 17327.

In use, the protective cover is removed from the flexible and rigid substrates 10 and 15 to expose the electrodes 13 and 14. The rigid substrate 15 is then manually placed on top of the flexible substrate 10 with the pairs of electrodes 13 and 14 in alignment. The adhesive between the substrates 10 and 15 and electrodes 13 and 14 will bond the two substrates 10 and 15 together and is of sufficient strength to prevent the substrates from inadvertently coming apart during use. However, the strength of the adhesive is not so strong so as to prevent the flexible and rigid substrates 10 and 15 from being manually separated without damaging the electrodes 13 and 14.

Once the flexible substrate or diaper to which it is fixed has been soiled, it is envisaged that the second part of the circuit, housed by the rigid substrate 15 is able to be separated or removed from the flexible substrate 10 so that the rigid housing can be reused by being fitted to a fresh diaper. The flexible substrate 10 forming part of a diaper and first part 12 of the circuit can be disposed or washed. In order to reduce costs, it is envisaged that the second part 17 of the circuit that is intended to be reused includes the more costly components of the electric circuit.

The second embodiment shown in the Figures 4 to 6 is substantially the same as the embodiment shown in Figures 1 to 3. The only substantial difference between the embodiments is that the rigid substrate 15 of the embodiment shown in figures 1 to 3 has been substituted with a flexible substrate 16 which like the rigid substrate 15 of the first embodiment includes all of the components of the circuit that are intended to be reused. The substrates 10 and 16 are also bonded together with the pairs of contact surfaces 13 and 14 in electrical connection using a suitable adhesive.

Embodiments include the following numbered clauses:
1. A device that may be worn by a person or fitted to an apparatus for monitoring, sensing or controlling one or more parameters such as, but by no means limited to, moisture, temperature, heart rate, respiration rate, strain, movement, light intensity, sound, pressure or impact forces, or an electrical function, wherein the device includes:
   a) a flexible substrate that can be worn by a person or attached to an apparatus and, wherein the flexible substrate requires disposal or replacement with a fresh flexible substrate or requires maintenance that prevents continuous use of the flexible substrate; and
   b) an electrical circuit having separate first and second parts, wherein the first part is carried by the flexible substrate and the first and second parts can be connected together to form an electrical connection therebetween and thereafter disconnected to allow the parts to be reused or disposed as desired, and wherein one or both of the first and second parts includes any one or combination of a controller for controlling a parameter 1 a monitor for monitoring a parameter or a sensor for sensing a parameter.
2. The device according to clause 1, wherein the first part of the circuit is directly attached to the flexible substrate.
3. The device according to clause 1 or 2, wherein the first part of the circuit is able to flex such that both the flexible substrate and the first part of the circuit is able to be flexed.
4. The device according to any one of clauses 1 to 3, wherein the first part of the circuit includes printed circuitry that is formed on or embedded in the flexible substrate.
5. The device according to any one of clauses 1 to 4, wherein the first part of the circuit includes an electrical conductive material that is on or embedded in the flexible substrate.
6. The device according to clause 5, wherein the conductive material is a filament or yarn that has been woven, knitted or incorporated in the flexible substrate by way of a layered structure.
7. The device according to any one of clauses 1 to 6, wherein the second part of the circuit is directly or indirectly connected to a second flexible substrate.
8. The device according to any one of clauses 1 to 7, wherein the second part of the circuit is housed by a rigid enclosure.
9. The device according to any one of clauses 1 to 7, wherein the second part of the circuit is carried or supported on another flexible substrate.
10. The device according to any one of clauses 1 to 9, wherein the first and second parts are able to be manually connected and disconnected.
11. The device according to clause 10, wherein the first and second parts are held together in electrical connection by any one or a combination of clips, clasps, Velcro, any other co-operating male and female members, or a releasable conductive adhesive.
12. The device according to clause 11, wherein the adhesive resists accidental separation of the first and second parts of the circuit yet allows the first and second parts to be manually separated.
13. The device according to any one of clauses 1 to 12, wherein the first part of the circuit includes a first pair of contact surfaces and that the second part of the circuit includes a second pair of co-operating contact surfaces, and that the first and second pairs of the contact surfaces can be manually aligned and placed together to facilitate electrical connection therebetween.
14. The device according to clause 13, wherein an electrical connective adhesive is located between and forms an electrical path between the first and second pairs of contact surfaces.
15. The device according to clause 14, wherein the conductive adhesive is pre-applied to one of the contact surfaces before electrical connection and that a removal protective tab is positioned to cover the adhesive on the contact surfaces so as to prevent contamination thereof prior to use.
16. The device according to any one of clauses 13 to 15, wherein the contact surfaces of the first or second parts have a total surface area of greater than or equal to 1 square millimetre.
17. The device according to any one of clauses 1 to 16, wherein the electrical circuit includes any one or a combination of:
   - an electrical power source for powering the circuit;
   - a processing unit for receiving information from the controller, monitor or sensor on the parameter being monitored, sensed or controlled and for comparing the information on the parameter to a pre-selected value or set of values of the parameter;
   - a controller interface for allowing the on and off operation of the power source or control another operational function of the device;
   - an output system for sending processed or unprocessed signals or for signalling when information on the parameter being controlled, monitored or sensed equals, exceeds or is less than a pre-selected value of the parameter being monitored, sensed or controlled.
18. The device according to clause 17, wherein the processing unit is a processing chip or micro controller.
19. The device according to clause 17, wherein the control interface is a switch.
20. The device according to any one of clauses 17 to 19, wherein the output system includes either one or combination of: a transmitter that transmits a signal to a receiver; or an indicator that emits light and/or sound.
21. The device according to any one of clauses 18 to 20, wherein the output system transmits a signal to a centralized station or care manager who may be caring for one or any number of patients.
22. The device according to any one of clauses 1 to 19, wherein the flexible substrate on which the first part of the circuit is carried is incorporated in, or forms part of, a garment or a textile for making a garment.
23. The device according to clause 20, wherein the garment is a disposable diaper, pad, incontinency sanitary product or wound dressing, and the parameter being monitored, sensed or controlled is moisture and the first part of the circuit carried by the flexible substrate forms part of the garment and can be disposed and the second part of the circuit can be reused by removing it from the used garment and connecting it to a fresh garment.

A person skilled in the art of the present invention will appreciate that many modifications and variations may be made to the preferred embodiments described above without departing from the spirit or scope of the present invention.

For example, the first part of the circuit mounted or embedded in the flexible substrate may include, but is by no means limited to, any one or combination of the following electrical components:
- a thermocouple for sensing temperature;
- a strain gauge for monitoring movement or impact forces;
- a light or sound sensor;
- a switch or any other form of control interface that allows adjustment; and
- suitable couplings for sensing heart rate or respiration rate of patient or athlete.

## Claims

1. A device that may be worn by a person or fitted to an apparatus for monitoring, sensing or controlling one or more parameters such as, but by no means limited to, moisture, temperature, heart rate, respiration rate, strain, movement, light intensity, sound, pressure or impact forces, or an electrical function, wherein the device includes:
a) a flexible substrate that can be worn by a person or attached to an apparatus and, wherein the flexible substrate requires disposal or replacement with a fresh flexible substrate or requires maintenance that prevents continuous use of the flexible substrate; and
b) an electrical circuit having separate first and second parts and one or both of the first and second parts includes any one or a combination of a controller for controlling a parameter, a monitor for monitoring a parameter or a sensor for sensing a parameter, wherein the first part is carried by the flexible substrate and includes a first pair of contact surfaces and the second part includes a second pair of contact surfaces and the first and second pairs of contact surfaces can be connected together to form an electrical connection therebetween and thereafter disconnected to allow the parts to be reused or disposed as desired, and wherein an electrical conductive adhesive is present on the contact surfaces of either one or both of the first and second pairs and electrical current can be conducted therethrough when the first and second pairs are connected.

2. The device according to claim 1, wherein the first part of the circuit is directly attached to the flexible substrate, and preferably
the first part of the circuit is able to flex such that both the flexible substrate and the first part of the circuit is able to be flexed, and preferably
the first part of the circuit includes printed circuitry that is formed on or embedded in the flexible substrate.

3. The device according to claim 1 or 2, wherein the first part of the circuit includes an electrical conductive material that is on or embedded in the flexible substrate, and preferably
the conductive material is a filament or yarn that has been woven, knitted or incorporated in the flexible substrate by way of a layered structure.

4. The device according to any one of claims 1 to 3, wherein the second part of the circuit is directly or indirectly connected to a second flexible substrate, and preferably
the second part of the circuit is housed by a rigid enclosure.

5. The device according to any one of claims 1 to 3, wherein the second part of the circuit is carried or supported on another flexible substrate.

6. The device according to any one of claims 1 to 9, wherein the first and second parts are able to be manually connected and disconnected, and perferably
the first and second parts are, in addition to the conductive adhesive, held together in electrical connection by any one or a combination of clips, clasps, Velcro™, any other co-operating male and female members, and preferably
the adhesive resists accidental separation of the first and second parts of the circuit yet allows the first and second parts to be manually separated, and preferably the first and second pairs of the contact surfaces can be manually aligned and placed together to facilitate electrical connection therebetween.

7. The device according to any one of the preceding claims, wherein an electrical conductive adhesive is pre-applied to either one or both of the first and second pairs of contact surfaces, and preferably
a removable protective tab is positioned to cover the adhesive on the contact surfaces so as to prevent contamination thereof prior to use, and preferably
the contact surfaces of the first or second parts have a total surface area of greater than or equal to 1 square millimetre.

8. The device according to any one of claims 1 to 7, wherein the electrical circuit includes any one or a combination of:
• an electrical power source for powering the circuit;
• a processing unit for receiving information from the controller, monitor or sensor on the parameter being monitored, sensed or controlled and for comparing the information on the parameter to a pre-selected value or set of values of the parameter, wherein the processing unit is preferably a processing chip or micro controller;
• a controller interface for allowing the on and off operation of the power source or control another operational function of the device, wherein the control interface is preferably a switch;
• an output system for sending processed or unprocessed signals or for signalling when information on the parameter being controlled, monitored or sensed equals, exceeds or is less than a pre-selected value of the parameter being monitored, sensed or controlled, wherein the output system preferably includes either one or combination of: a transmitter that transmits a signal to a receiver; or an indicator that emits light and/or sound; and preferably the output system transmits a signal to a centralized station or care manager who may be caring for one or any number of patients.

9. The device according to any one of claims 1 to 8, wherein the flexible substrate on which the first part of the circuit is carried is incorporated in, or forms part of, a garment or a textile for making a garment, and preferably
the garment is a disposable diaper, pad, incontinency sanitary product or wound dressing, and the parameter being monitored, sensed or controlled is moisture and the first part of the circuit carried by the flexible substrate forms part of the garment and can be disposed and the second part of the circuit can be reused by removing it from the used garment and connecting it to a fresh garment.

10. A device that may be worn by a person or fitted to an apparatus for monitoring, sensing or controlling one or more parameters such as, but by no means limited to, moisture, temperature, heart rate, respiration rate, strain, movement, light intensity, sound, pressure or impact forces, or an electrical function, wherein the device includes:
a) a flexible substrate that can be worn by a person or attached to an apparatus and, wherein the flexible substrate requires disposal or replacement with a fresh flexible substrate or requires maintenance that prevents continuous use of the flexible substrate; and
b) a first part of an electrical circuit that is connectable to a second part of the electrical circuit, wherein the first part is attached to the flexible substrate and the first part includes a pair of contact surfaces for connection with a co-operating pair of contact surfaces of the second part, and wherein an electrical conductive adhesive is pre-applied to the contact surfaces of the first part such that the first and second parts can be connected together to form an electrical connection therebetween and thereafter disconnected to allow the first part of the circuit to be disposed as desired together with the substrate.

11. The device according to claim 10, wherein the first part of the circuit is able to flex such that both the flexible substrate and the first part of the circuit is able to be flexed, and preferably
the first part of the circuit includes printed circuitry that is formed on or embedded in the flexible substrate, and preferably
the first part of the circuit includes an electrical conductive material that is on or embedded in the flexible substrate.

12. The device according to claim 11, wherein the conductive material is a filament or yarn that has been woven, knitted or incorporated in the flexible substrate by way of a layered structure.

13. The device according to any one of claims 10 to 12, wherein prior to connection of the first and second parts a removable protective tab is positioned to cover the adhesive on the contact surfaces so as to prevent contamination thereof prior to use, and preferably
the contact surfaces of the first part have a total surface area of greater than or equal to 1 square millimetre.

14. The device according to any one of claims 10 to 13, wherein the flexible substrate on which the first part of the circuit is attached is incorporated in, or forms part of, a garment or a textile for making a garment.

15. The device according to claim 14, wherein the garment is a disposable diaper, pad, incontinency sanitary product or wound dressing, and the parameter being monitored, sensed or controlled is moisture and the first part of the circuit carried by the flexible substrate forms part of the garment and can be disposed and the second part of the circuit can be reused by removing it from the used garment and connecting it to a fresh garment.
